# EUROPEAN PATENT SPECIFICATION

(11) **EP 4 208 228 B1**
(45) Date of publication and mention of the grant of the patent: **01.10.2025**
(21) Application number: 21773475.5
(22) Date of filing: 31.08.2021
(51) Int. Cl.: A61M 11/06, A61M 15/00, A61M 15/08, B05B 11/02, A61M 11/00

(54) **DRUG DELIVERY DEVICES WITH A MECHANICAL STOP**
ARZNEIMITTELABGABEVORRICHTUNGEN MIT MECHANISCHEM ANSCHLAG
DISPOSITIFS D'ADMINISTRATION DE MÉDICAMENT À BUTÉE MÉCANIQUE

(30) Priority: 01.09.2020 US 202063073026 P
(43) Date of publication of application: 12.07.2023
(73) Proprietor: Janssen Pharmaceutica NV, 2340 Beerse (BE)
(72) Inventor: RAMOS, David, Orange Park, Florida 32065 (US); POPLI, Shagun, Milpitas, California 95035 (US); CANNAMELA, Michael, New York 10001 (US); CASSEBEE, Jimmy, Vinh, Hoang, Milpitas, California 95035 (US); YAN, Hong, Milpitas, California 95035 (US); PÉREZ, Dolores, Easton, Pennsylvania 18045 (US); WANG, Jingli, Milpitas, California 95035 (US); LARSON, Chaley John, Horsham, Pennsylvania 19044 (US); SCRIMGEOUR, Ian, Dunbar EH42 ILT (GB); HUBERT, Emma, Louise, Milpitas, California 95035 (US); KAPIL, Monica A., San Jose, California 95127 (US); VESOLE, Steven, M., Milpitas, California 95035 (US)
(74) Representative: Carpmaels & Ransford LLP
(86) International application number: PCT/EP2021/074052
(87) International publication number: WO 2022/049085

(56) References cited:
- EP-A1- 1 377 336
- EP-A1- 3 278 822
- CA-A1- 2 040 053
- US-A1- 2002 010 428
- US-A1- 2010 258 118

## Description

### FIELD

The present disclosure relates generally to drug delivery devices with a mechanical stop and drug products utilizing the same.

### BACKGROUND

EP 1377336 A1 and US 2002/010428 A1 discuss a drug delivery system for administering present doses of a substance such as a drug, vaccine or the like. The drug delivery system includes a holder and a pre-filled drug container such as a syringe which is securely retained in the holder. The drug container includes a barrel for containing the substance, a movable stopper situated within the barrel, and a blunt end having an opening through which the substance within the barrel can be expelled. The holder includes a distal portion and a proximal portion, each configured to accommodate the drug container, with the distal portion being able to be assembled to the proximal portion, which acts as a plunger rod during activation of the delivery system. A plurality of slots are provided for controlling the delivery of a substance and extend axially along at least one portion of the holder whereby upon activation of the system, the portions of the holder move towards one another upon the application of a minimum force and the stopper moves a preselected axial distance to expel at least a portion of the substance form the drug container. One application is to a nasal drug container, where approximately equal doses of the substance are desired to be administered to each nostril of the patient.
EP 3278822 A1 discusses a delivery device for delivery of a medicament to a user including a container defining a reservoir storing the medicament, a body in which the container is received, a spray nozzle arranged on one of the body and the container for receiving medicament expelled from the container, and a pusher actuatable for pushing the medicament from the container reservoir through the nozzle, said pusher being movable from a rest position to a fully activated position relative to said body in response to an actuating force. The assembly is particularly applicable to nasal syringes where it is desirable to deliver the medicament in the form of a spray.
CA 2040053 A1 discusses a device for the transnasal or oral administration of drugs or similar media, comprising a finger-like applicator which has a spray nozzle disposed at its free end and includes a cylinder space for accommodating the medium to be administered, said medium being applicable by means of a piston from the cylinder space through the spray nozzle. The spray nozzle is formed by a membrane-like seal having a fluid passage which opens under pressure.
US 2010/258118 A1 discusses an inhaler devices and bespoke pharmaceutical dry powder composition to be dispensed using such inhaler devices for pulmonary administration. In particular, the present invention relates to the provision of passive inhaler devices and dry powder compositions which are specifically formulated and prepared to be efficiently dispensed by such devices to reproducibly achieve a high delivered dose of the pharmaceutically active agent.

There are many different ways in which a drug can be administered to a user. Depending on the drug, intranasal drug delivery can be one of the most effective ways to achieve desired clinical benefits in a timely manner and in a manner that is convenient and comfortable for a patient.

Intranasal drug administration is a non-invasive route for drug delivery. Since the nasal mucosa offers numerous benefits as a target tissue for drug delivery, a wide variety of drugs may be administered by intranasal systemic action. Moreover, intranasal drug delivery can avoid the risks and discomfort associated with other routes of drug delivery, such as intravenous drug delivery, and can allow for easy self-administration.

Generally, to maximize the efficacy of the drug through intranasal administration, the majority volume of the aerosolized dose of the drug needs to reach the correct region of the nasal cavity. As such, additional measures may need to be taken for effective intranasal drug delivery. For example, the user may need to have a clear nostril, tilt their head back at approximately 45°, close the opposite nostril, and then sniff gently while the dose of drug is administered. In order to coordinate these measures, and given that nasal administration is intimate, self-administration by the user may be desired. Further, due to the nasal cycle (alternating physiological partial congestion of the nasal turbinate to facilitate nasal function) or pathological congestion, one nostril is likely to provide a more effective drug delivery route than the other nostril at any given time. As such, it is desired that an equal dose of the drug be delivered to each nostril of the user to inhibit under-dosing of the drug.

Dual-dose intranasal drug delivery devices are available that are designed for self-administration of two distinct aerosolized sprays, one for each nostril, that together constitute one dose of drug. These devices require a series of operational steps that the user needs to properly carry out to effect optimal drug delivery through self-administration.

Accordingly, there remains a need for improved nasal drug delivery devices.

### SUMMARY

In general, drug delivery devices with a mechanical stop, drug products utilizing the same, and methods of using drug delivery devices with a mechanical stop are provided. The invention is defined by the appended claims.

In one aspect, a drug delivery system is provided that in one embodiment includes a dispensing head including a tip configured to be positioned in a nose of a patient. The tip has an opening therein. The drug delivery system also includes a drug holder containing a drug therein, a first stop member, and a second stop member. The drug holder is configured to move a first amount relative to the dispensing head in a first stage of operation in which the drug is delivered from the drug holder and out of the opening, the drug holder is configured to move a second amount relative to the dispensing head in a second stage of operation in which the drug is delivered from the drug holder and out of the opening, the first stop member is configured to mechanically stop movement of the drug holder relative to the dispensing head between the first and second stages of operation, and the second stop member is configured to mechanically stop movement of the drug holder relative to the dispensing head after the second stage of operation.

The drug delivery system can vary in any number of ways. For example, the drug delivery system can also include an accessory that can include the first stop member, that can be configured to be releasably coupled to the drug holder during the first stage of operation, and that can be configured to be removed from the drug holder between the first and second stages of operation. The accessory can have an inner passageway extending therethrough, and the accessory can be configured to be releasably coupled to the drug holder during the first stage of operation with the drug holder extending through the inner passageway. The drug delivery system can also include a body that includes the second stop member, the second stop member can include an exterior surface of the body, the drug holder can be configured to move the first amount relative to the body in the first stage of operation, and the drug holder can be configured to move the second amount relative to the body in the second stage of operation.

For another example, the drug delivery system can also include a body that includes the first and second stop members, the first stop member can include a first exterior surface of the body, the second stop member can include a second exterior surface of the body that is proximal to the first exterior surface, the drug holder can be configured to move the first amount proximally relative to the body in the first stage of operation, and the drug holder can be configured to move the second amount proximally relative to the body in the second stage of operation.

For yet another example, the drug holder can include an elongate body with a flange extending radially outward from the elongate body, and the flange can be releasably attached to the elongate body with a plurality of frangible legs. The plurality of frangible legs can each be configured to break during the first stage of operation. The drug delivery system can also include a centerpiece that includes the first stop member, the drug delivery system can also include a body that includes the second stop member, the first stop member can include a protrusion configured to engage the flange, and the second stop member can include an interior surface of the body configured to engage a surface of the drug holder. The drug delivery system can also include a body that includes the second stop member, the drug holder can include a second flange extending radially outward from the elongate body, and the second flange can be releasably attached to the elongate body with a second plurality of frangible legs that are each configured to break during the second stage of operation.

For still another example, the drug holder can have first and second grooves formed therein, the drug delivery system can also include a body having first and second protrusions extending radially inward therefrom, the first stop member can include the first groove and the first protrusion, and the second stop member can include the second groove and the second protrusion. For another example, the drug holder can include a frangible tab extending radially outward therefrom, the drug delivery system can include a body including a protrusion extending radially inward therefrom that defines the first stop member, and the body can include an interior surface that defines the second stop member. For yet another example, the drug holder can be configured to be pushed in an axial direction toward the dispensing head in each of the first and second stages of operation, the drug holder can have a helical groove formed therein, and the drug delivery system can also include a cam configured to slide within the helical groove in response to the pushing of the drug holder, thereby causing the drug holder to rotate relative to the dispensing head. For still another example, the drug holder can be configured to be pushed in a longitudinal direction toward the dispensing head in each of the first and second stages of operation, the drug holder can have a protrusion extending therefrom, the drug delivery system can also include a body having a track formed therein, the protrusion can be configured to slide longitudinally within the track in response to the pushing of the drug holder, thereby causing the protrusion of the drug to slide axially in the track, the drug holder can be configured to be rotated relative to the dispensing head between the first and second stages of operation, and the protrusion can be configured to slide axially within the track in response to the rotation of the drug holder. For another example, the drug delivery system can also include a body with a sleeve disposed therein that has the drug holder fixed thereto, the drug delivery system can also include an actuator configured to be actuated to cause the drug to be delivered out of the opening, the drug delivery system can also include a push spring configured to push the sleeve and the drug holder proximally in response to the actuation of the actuator, the first stop member can include a first surface of the sleeve, and the second stop member can include a second surface of the sleeve. For still another example, the drug delivery system can include a first actuator that can include the first stop member and that can be configured to be actuated to cause the drug to be delivered out of the opening in the first stage of operation, the drug delivery system can include a second actuator that can include the second stop member and that can be configured to be actuated to cause the drug to be delivered out of the opening in the first stage of operation, the drug delivery system can include a proximal magnet, and the drug delivery system can include a distal magnet configured to be drawn proximally toward the proximal magnet in response to the actuation of the first actuator and in response to the actuation of the second actuator. For yet another example, the drug delivery system can include a viewing window configured to allow a user to visualize whether or not each of the first and second stages of operation have occurred. For another example, the drug can be one of ketamine, esketamine, naloxone, and sumatriptan.

In another aspect, a drug product is provided that in one embodiment includes a drug product disposed in a drug delivery system. The drug delivery system includes a dispensing head including a tip configured to be positioned in a nose of a patient. The tip has an opening therein. The drug delivery system also includes a drug holder containing the drug product therein, a first stop member, and a second stop member. The drug holder is configured to move a first amount relative to the dispensing head in a first stage of operation in which the drug product is delivered from the drug holder and out of the opening, the drug holder is configured to move a second amount relative to the dispensing head in a second stage of operation in which the drug product is delivered from the drug holder and out of the opening, the first stop member is configured to mechanically stop movement of the drug holder relative to the dispensing head between the first and second stages of operation, and the second stop member is configured to mechanically stop movement of the drug holder relative to the dispensing head after the second stage of operation. The drug product is one of ketamine, esketamine, naloxone, and sumatriptan. The drug delivery system can have any number of variations.

For another example, a body of the drug delivery device can include the first and second mechanical stop members, the first mechanical stop member can include a first exterior surface of the body, and the second mechanical stop member can include a second exterior surface of the body that is proximal to the first exterior surface. The body of the drug delivery device can be held by hand in a first hand position relative to the body in the first stage of operation, and the body of the drug delivery device can be held by hand in a second, different hand position relative to the body in the second stage of operation.

For yet another example, the drug holder can include an elongate body with a flange extending radially outward from the elongate body, the flange can be releasably attached to the elongate body with a plurality of frangible legs, and actuating the drug delivery device in the first stage of operation can cause the frangible legs to break. A centerpiece disposed in the drug delivery device can define the first mechanical stop member. The drug holder can include a second flange extending radially outward from the elongate body, the second flange can be releasably attached to the elongate body with a second plurality of frangible legs, and actuating the drug delivery device in the second stage of operation can cause the second frangible legs to break.

For yet another example, the drug holder can have first and second grooves formed therein, a body can have first and second protrusions extending radially inward therefrom, the first mechanical stop member can include the first groove and the first protrusion, and the second mechanical stop member can include the second groove and the second protrusion. For another example, the drug holder can include a frangible tab extending radially outward therefrom, and actuating the drug delivery device in the first stage of operation can cause the frangible tab to break.

For still another example, actuating the drug delivery device in the first stage of operation can include pushing the drug holder toward the dispensing head in each of the first and second stages of operation. The drug holder can have a helical groove formed therein, and a cam can slide within the helical groove in response to the pushing of the drug holder, thereby causing the drug holder to rotate relative to the dispensing head. A body of the drug delivery device can have a track formed therein, and a protrusion extending from the drug holder can slide within the track in response to the pushing of the drug holder. The method can also include rotating the drug holder relative to the dispensing head between the first and second stages of operation, and the protrusion can slide axially within the track in response to the rotation of the drug holder.

For still another example, the drug delivery device can include a body with a sleeve disposed therein that has the drug holder fixed thereto, the drug delivery device can include a push spring that pushes the sleeve and the drug holder proximally in response to each of the actuations, the first mechanical stop member can include a first surface of the sleeve, and the second mechanical stop member can include a second surface of the sleeve. For another example, actuating the drug delivery device in the first stage of operation can cause a first magnet to be drawn proximally toward a second magnet, and actuating the drug delivery device in the second stage of operation again can cause the first magnet to be drawn proximally toward the second magnet. For yet another example, the drug can be one of ketamine, esketamine, naloxone, and sumatriptan.

### BRIEF DESCRIPTION OF DRAWINGS

The present invention is described by way of reference to the accompanying figures which are as follows:
FIG. 1 is a block diagram of one embodiment of a drug delivery device;
FIG. 2 is a side view of one embodiment of the drug delivery device of FIG. 1;
FIG. 3 is a side cross-sectional view of a portion of the drug delivery device of FIG. 2;
FIG. 4 is a side view of the drug delivery device of FIG. 2 coupled to one embodiment of an accessory;
FIG. 5 is a perspective view of the accessory of FIG. 4;
FIG. 6 is a side view of a drug holder of the drug delivery device of FIG. 2;
FIG. 7 is a side view of the drug delivery device and accessory of FIG. 4 after a first drug delivery from the drug delivery device;
FIG. 8 is a side view of the drug delivery device of FIG. 7 with the accessory removed therefrom;
FIG. 9 is a side view of the drug delivery device of FIG. 8 after a second drug delivery from the drug delivery device;
FIG. 10 is a side schematic view of a hand holding the drug delivery device of FIG. 2;
FIG. 11 is a side schematic view of the drug delivery device of FIG. 10 after a first drug delivery from the drug delivery device;
FIG. 12 is a side schematic view of the drug delivery device of FIG. 11 after a second drug delivery from the drug delivery device;
FIG. 13 is a partial, side cross-sectional view of another embodiment of the drug delivery device of FIG. 1;
FIG. 14 is a perspective view of a drug holder of the drug delivery device of FIG. 13;
FIG. 15 is a top view of the drug holder of FIG. 14;
FIG. 16 is a perspective view of a centerpiece of the drug delivery device of FIG. 13;
FIG. 17 is a top view of the centerpiece of FIG. 16;
FIG. 18 is a perspective view of another embodiment of a drug holder;
FIG. 19 is a side partial cross-sectional view of another embodiment of the drug delivery device of FIG. 1;
FIG. 20 is an exploded partial view of yet another embodiment of the drug delivery device of FIG. 1;
FIG. 21 is a side partial transparent view of still another embodiment of the drug delivery device of FIG. 1;
FIG. 22 is a side view of a portion of a drug holder of the drug delivery device of FIG. 21;
FIG. 23 is a side view of yet another embodiment of the drug delivery device of FIG. 1;
FIG. 24 is a side partial cross-sectional view of still another embodiment of the drug delivery device of FIG. 1;
FIG. 25 is a side partial cross-sectional view of the drug delivery device of FIG. 24 after a first drug delivery from the drug delivery device;
FIG. 26 is a side partial cross-sectional view of the drug delivery device of FIG. 25 after a second drug delivery from the drug delivery device;
FIG. 27 is a side partial cross-sectional view of another embodiment of the drug delivery device of FIG. 1;
FIG. 28 is a side partial cross-sectional view of the drug delivery device of FIG. 27 during a first drug delivery from the drug delivery device;
FIG. 29 is a side partial cross-sectional view of the drug delivery device of FIG. 24 after a first drug delivery from the drug delivery device;
FIG. 30 is a side partial cross-sectional view of the drug delivery device of FIG. 29 during a second drug delivery from the drug delivery device;
FIG. 31 is a side schematic partial view of another embodiment of the drug delivery device of FIG. 1; and
FIG. 32 is a side cross-sectional view of another embodiment of a drug holder.

### DETAILED DESCRIPTION

Certain exemplary embodiments will now be described to provide an overall understanding of the principles of the structure, function, manufacture, and use of the devices, systems, and methods disclosed herein. One or more examples of these embodiments are illustrated in the accompanying drawings. A person skilled in the art will understand that the devices, systems, and methods specifically described herein and illustrated in the accompanying drawings are non-limiting exemplary embodiments and that the scope of the present invention is defined solely by the claims. The features illustrated or described in connection with one exemplary embodiment may be combined with the features of other embodiments. Such modifications and variations are intended to be included within the scope of the present invention.

Further, in the present disclosure, like-named components of the embodiments generally have similar features, and thus within a particular embodiment each feature of each like-named component is not necessarily fully elaborated upon. Additionally, to the extent that linear or circular dimensions are used in the description of the disclosed systems, devices, and methods, such dimensions are not intended to limit the types of shapes that can be used in conjunction with such systems, devices, and methods. A person skilled in the art will recognize that an equivalent to such linear and circular dimensions can easily be determined for any geometric shape. A person skilled in the art will appreciate that a dimension may not be a precise value but nevertheless be considered to be at about that value due to any number of factors such as manufacturing tolerances and sensitivity of measurement equipment. Sizes and shapes of the systems and devices, and the components thereof, can depend at least on the size and shape of components with which the systems and devices will be used.

Various exemplary drug delivery devices with a mechanical stop, drug products utilizing the same, and methods of using drug delivery devices with a mechanical stop are provided. In general, a nasal drug delivery device is configured to dispense therefrom first and second doses of a drug therefrom into a nose. The drug delivery device includes a mechanical stop configured to provide a pause between the delivery of the first and second doses. During the pause, a user of the drug delivery device can move the drug delivery device from one nostril, into which the first dose was sprayed, to another nostril, into which the second dose can be sprayed. The mechanical stop is configured to hold the drug delivery device in a static delivery state such that the user can choose a time to begin delivery of the second dose, e.g., when to actuate the drug delivery device for a second time.

The drug to be delivered using a drug delivery device as described herein can be any of a variety of drugs. Examples of drugs that can be delivered using a drug delivery device as described herein include antibodies (such as monoclonal antibodies), hormones, antitoxins, substances for the control of pain, substances for the control of thrombosis, substances for the control of infection, peptides, proteins, human insulin or a human insulin analogue or derivative, polysaccharide, DNA, RNA, enzymes, oligonucleotides, antiallergics, antihistamines, anti-inflammatories, corticosteroids, disease modifying anti-rheumatic drugs, erythropoietin, and vaccines. Examples of drugs that can be delivered using a drug delivery device as described herein include ketamine (e.g., Ketalar^{®}), esketamine (e.g., Spravato^{®}, Ketanest^{®}, and Ketanest-S^{®}), naloxone (e.g., Narcan^{®}), and sumatriptan (e.g., Imitrex^{®}).

A drug delivery device configured to expel a drug into a nose of a patient and to drive delivery of the drug using a propellant can have a variety of configurations. In general, the drug delivery device is configured to deliver a drug to a patient, where the drug is provided in a defined dosage form within the drug delivery device.

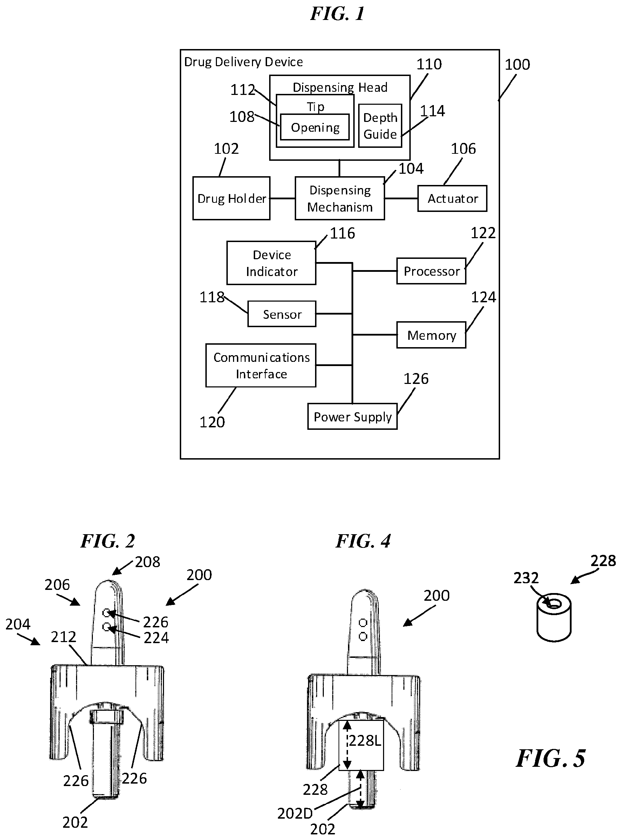

FIG. 1 illustrates one embodiment of a drug delivery device 100 configured to expel a drug into a nose of a patient. As will be appreciated by a person skilled in the art, the drug delivery device 100 can include different features in different embodiments depending upon various requirements, such as the type of drug, typical dosage(s) of the drug, safety requirements in various jurisdictions, whether the device is powered, etc.

The drug delivery device 100 includes a drug holder 102 configured to contain a drug therein for delivery from the device 100 to a patient. The drug holder 102 can have a variety of configurations, such as a cartridge, a vial, a blow-fill-seal (BFS) capsule, a blister pack, etc. In an exemplary embodiment, the drug holder 102 is a vial. An exemplary vial is formed of one or more materials, e.g., glass, polymer(s), etc. In some embodiments, a vial can be formed of glass. In other embodiments, a vial can be formed of one or more polymers. In yet other embodiments, different portions of a vial can be formed of different materials.

The drug delivery device 100 also includes a dispensing mechanism 104 that is operatively coupled to the drug holder 102 and configured to drive the drug out of device 100 from the drug holder 102. The dispensing mechanism 104 can have a variety of configurations. For example, the dispensing mechanism 104 can include a plunger configured to push the drug out of the drug holder 102. For another example, the dispensing mechanism 104 can include a piston, pin, and/or a needle configured to pierce through or puncture a seal member of the drug holder 102 in embodiments in which the drug holder 102 includes a pierceable or puncturable seal member.

The drug delivery device 100 also includes an actuator 106 configured to be actuated by a user to cause the dispensing mechanism 104 to begin delivering a dose of the drug through an opening or nozzle 108 in the drug delivery device 100. In an exemplary embodiment, the drug delivery device 100 is configured to be self-administered such that the user who actuates the actuator 106 is the patient receiving the drug from the drug delivery device 100. The actuator 106 can have a variety of configurations, as discussed further below. For example, the actuator 106 can include a pressable button. For another example, the actuator 106 can include a movable switch. For yet another example, the actuator 106 can include a squeezable body of the drug holder 102.

The opening 108 through which the drug exits the drug delivery device 100 is formed in a dispensing head 110 of the drug delivery device 100 in a tip 112 of the dispensing head 110. The tip 112 is configured to be inserted into a nostril of a patient. In an exemplary embodiment, the tip 112 is configured to be inserted into a first nostril of the patient during a first stage of operation of the drug delivery device 100 and into a second nostril of the patient during a second stage of operation of the drug delivery device 100. The first and second stages of operation involve two separate actuations of the actuator 106, a first actuation corresponding to a first dose of the drug being delivered and a second actuation corresponding to a second dose of the drug being delivered. The dispensing head 110 includes a depth guide 114 configured to contact skin of the patient, e.g., between the patient's first and second nostrils, such that a longitudinal axis of the dispensing head 110 is substantially aligned with a longitudinal axis of the nostril in which the tip 112 is inserted. A person skilled in the art will appreciate that the longitudinal axes may not be precisely aligned but nevertheless be considered to be substantially aligned due to any number of factors, such as manufacturing tolerances and sensitivity of measurement equipment.

In an exemplary embodiment, the dispensing head 110 has a tapered shape in which the dispensing head 110 has a smaller diameter at its distal end than at its proximal end where the opening 108 is located. The opening 108 having a relatively small diameter facilitates spray of the drug out of the opening 108, as will be appreciated by a person skilled in the art. A spray chamber through which the drug is configured to pass before exiting the opening 108 is located within a proximal portion of the tapered dispensing head 110, distal to the opening 108. When the drug passes through the spray chamber at speed, the spray chamber facilitates production of a fine mist that exits through the opening 108 with a consistent spray pattern.

**In** some embodiments, the dispensing head 110 can include two tips 112 each having an opening 108 therein such that the drug delivery device 100 is configured to simultaneously deliver doses of drug into two nostrils in response to a single actuation of the actuator 106.

The drug delivery device 100 also includes a device indicator 116 configured to present information to a user about a status of the drug delivery device 100 and/or the drug contained in the drug holder 102. The device indicator 116 can be a visual indicator, such as a display screen, one or more lights, one or more colored and/or numbered markings, etc. Alternatively or in addition, the device indicator 116 can be an audio indicator configured to provide sound.

The drug delivery device 100 also includes a sensor 118 configured to sense information relating to the drug delivery device 100 and/or the drug contained in the drug holder 102. Examples of information that the sensor 118 can sense include environmental conditions (e.g., temperature, humidity, geographic location, time, etc.). The drug delivery device 100 can also include a communications interface 120 configured to communicate externally data which has been gathered by the sensor 118 about the drug delivery device 100 and/or the drug contained in the drug holder 102, which may facilitate analysis regarding the patient's treatment, patient compliance, use of the drug delivery device 100, etc.

In embodiments in which the drug delivery device 100 includes one or more electrical components, e.g., the device indicator 116 (which in some embodiments can be powered while in other embodiments not be powered), the sensor 118, the communications interface 120, a processor 122, a memory 124, etc., the drug delivery device 100 includes a power supply 126 configured to deliver electrical power to the one or more electrical components of the drug delivery device 100. The power supply 126 can be a source of power which is integral to drug delivery device 100 and/or can be a mechanism configured to connect the drug delivery device 100 to an external source of power. The processor 122 is configured to receive gathered data from the sensor 118 and to cause the data to be stored in the memory 124, to be indicated on the device indicator 110, and/or to be communicated externally via the communications interface 120. The memory 124 is configured to store instructions that are configured to be executed by the processor 122 for the processor 122 to process information regarding the various electrical components with which the processor 122 is in communication.

As mentioned above, the drug delivery device 100 can include different features in different embodiments depending upon various requirements. For example, the drug delivery device 100 can omit any one or more of the depth guide 114, the device indicator 116, the sensor 118, the communications interface 120, the processor 122, the memory 124, and the power supply 126.

FIG. 2 illustrates an exemplary embodiment of the drug delivery device 100 of FIG. 1. FIG. 2 illustrates a drug delivery device 200 configured to expel a drug into a nose of a patient in two stages of operation. In a first stage of operation a first dose of the drug is delivered, and in a second stage of operation a second dose of the drug is delivered.

The drug delivery device 200 is generally configured and used similar to that discussed above regarding FIG. 1 and includes a drug holder 202 and a dispensing head 204 that includes a tip 206 and an opening 208. The tip 206 is configured to be inserted into a first nostril during a first stage of operation of the drug delivery device 200 and into a second nostril during a second stage of operation of the drug delivery device 200. The vial 202 thus defines an actuator configured to be actuated to cause drug delivery to begin. The drug in this illustrated embodiment is configured to be self-administered such that the user who actuates the actuator, e.g., the vial 202, is the person receiving the drug from the drug delivery device 200, although another person can actuate the device 200 for delivery into another person.

The drug holder 202 in this illustrated embodiment is a vial. The vial 202 includes a seal member at a proximal end thereof that is configured to provide a fluid tight seal such that the drug is contained in the vial 202 until the seal provided by the seal member is broken. The seal provided by the seal member can be broken in a variety of ways, such as by being pierced by a piston of the drug delivery device 200 as discussed further below. The seal member can have a variety of configurations, as will be appreciated by a person skilled in the art, such as by being a pierceable polymer septum or a foil layer. The seal member can be protected from accidental puncturing or piercing before intended use with a removable protective member or stopper, such as a tamper evident (TE) seal, etc. In some embodiments, the seal member can be omitted and instead a removable protective member or stopper can be provided that is removed just prior to use of the vial 202.

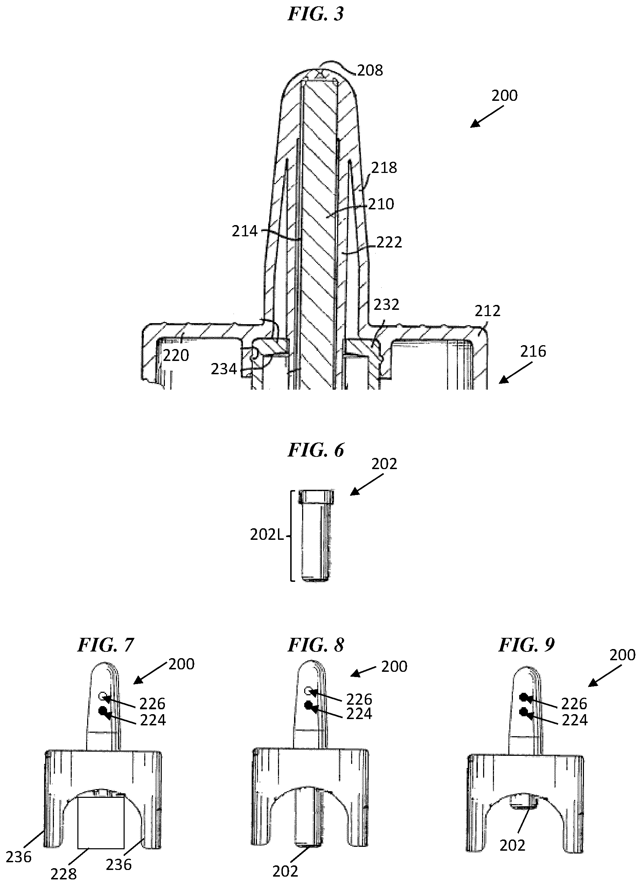

As shown in FIG. 3, the drug delivery device 200 includes a piston 210 that is fixed to the dispensing head 204. A distal end of the piston 210 is configured to puncture or pierce through the vial's seal member. The vial 202 can thus be fluid tight until the vial 202 is coupled to the drug delivery device 200, after which the drug contained in the vial 202 can flow out of the vial 202 for delivery. The distal end of the piston 210 can include a sharp edge, e.g., around a perimeter of the piston's distal end, to facilitate the puncturing or piercing of the vial's seal member or can otherwise include a feature configured to facilitate the puncturing or piercing, such as by having one or more sharp extensions extending distally therefrom. In some embodiments, the seal member can be omitted from the vial 202, in which case the piston 210 need not be configured to puncture or pierce through the vial's seal member. The drug delivery device 200 does not need to be primed after the vial 202 has been releasably coupled thereto and prior to actuation of the drug delivery device 200 to cause drug delivery, which may ease use of the drug delivery device 200 by a user.

The drug delivery device 200 includes a body 212 in the form of a casing. The vial 202 is configured to move relative to the body 212 to cause delivery of the drug from the drug delivery device 200 through the opening 208. The body 212 has the piston 210 fixed thereto. From an inner end face of the piston 210 emanates an outlet channel 214, which is guided in valve-free manner to the opening 208 leading into the open and which is formed by an atomizing nozzle. The outlet channel 214 becomes to be in fluid communication with a hollow interior of the vial 202 in response to the piston 210 puncturing or piercing the seal member of the vial 202.

The body 212 includes a handle portion 216 (partially shown in FIG. 3) configured to be handheld during use of the drug delivery device 200. As shown in FIG. 2, flattened sides of the handle portion 216 include two facing finger openings 226 configured to receive a thumb of a user's hand with the index and middle finger of this hand on either side of a discharge connection 218 of the handle portion 216 on an outside of the handle portion's end wall 220 or elsewhere on the handle portion 216. By moving together the thumb and the two other fingers the vial 202 can be displaced with respect to the piston 210 or the body 212 and the drug consequently sprayed out of the vial 202 through the discharge channel 214 and through the opening 208. The handle portion 216, as well as various other aspects of the drug delivery device 200, are further described in U.S. Pat. No. 4,946,069 entitled "Dispenser For Manually Discharging Flowable Media" issued Aug. 7, 1990.

The discharge connection 218 includes a piston sleeve 222 constructed in one piece therewith and radially spaced within the discharge connection 218. The piston sleeve 222 projects beyond the end wall 220 both outwards and inwards into the handle portion 216. The piston sleeve 222 carries the piston 210 inserted therein. For this purpose the rear end of the piston 210 is extended to a piston shaft constructed in one piece therewith and having substantially the same external cross-sections and which is set back with respect to the end face of the discharge connection 218 and with its associated end face engaging on an inner shoulder of the discharge connection 218 in such a way that a twisting device for the drug is formed between the end face and the discharge connection 218. In the vicinity of the piston 210, which only projects slightly beyond the piston sleeve 222, the outlet channel 214 is formed by a central longitudinal bore which, immediately behind the end of the piston sleeve 222 located at the piston 210, passes via a transverse bore into a main portion of the outlet channel 214, which is defined between an outer circumference of the piston shaft and an inner circumference of the piston sleeve 222 and which can, e.g., be formed by at least one longitudinal groove on the outer circumference of the piston shaft.

A base plate at a distal end of the vial 202 is curved inwards in such a way that it forms on its outside a thumb depression for the engagement of the thumb of the user's hand and on the inside has a correspondingly curved protuberance. A front end face of the piston 210 includes a depression that is shaped substantially identically to the protuberance and whose base face strikes against the cylinder base plate at the end of the second stage of operation, so that substantially no residual quantity of the drug to be discharged remains in the vial 202 after the second stage of operation. A person skilled in the art will appreciate that 100% of the drug may not have exited the vial 202, but the vial 202 can nevertheless be considered to have substantially no drug therein due to any number of factors, such as sensitivity of measurement equipment. The base plate is internal to the vial 202 and is thus obscured in FIG. 2. In other embodiments, the base plate is linear instead of curved.

A first actuation of the vial 202 causes the drug to be delivered into a first nostril into which the tip 206 has been inserted. A second actuation of the vial 202 causes the drug to be delivered into a second nostril into which the tip 206 has been inserted. As mentioned above, it is desired that an equal dose of the drug be delivered to each nostril to inhibit under-dosing of the drug.

Referring again to FIG. 2, the drug delivery device 200 includes first and second viewing windows 224, 226 formed in a side wall of the dispensing head 204. The first and second viewing windows 224, 226 are configured to be clearly visible to a user of the drug delivery device 200 when the drug delivery device 200 is held by hand. The viewing windows 224, 226 are circular in this illustrated embodiment but can have another shape, e.g., square, triangular, ovular, etc. The drug delivery device 200 includes only two viewing windows 224, 226 in this illustrated embodiment but can have another number of viewing windows. For example, third and fourth viewing windows can be formed in a side wall of the dispensing head 204 substantially 180° around a circumference of the dispensing head 204 that are configured and used similar to the first and second viewing windows 224, 226. The third and fourth viewing windows may facilitate a user's visualization of at least one pair of viewing windows, e.g., the first and second viewing windows 224, 226 or the third and fourth viewing windows, regardless of whether the user's right or left hand is holding the drug delivery device 200.

The viewing windows 224, 226 are configured to cooperate with a device indicator of the drug delivery device 200. The device indicator is configured to indicate to a user whether a first dose has been dispensed, e.g., whether the first stage of operation has occurred, and whether a second dose has been dispensed, e.g., whether the second stage of operation has occurred. The device indicator is configured to be visible through the viewing windows 224, 226 to indicate whether the first and second doses have been dispensed. **In** this way, the user can know a drug delivery status of the drug delivery device 200, including whether or not one or both of the first and second doses have been dispensed. The drug holder 202 in this illustrated embodiment has an amount of drug disposed therein for two doses, so indicating that two doses have been delivered thus indicates that substantially no drug remains in the drug holder 202. Some drugs, such as esketamine and other controlled substances, may be required to have drug delivery therefrom verified per the drug's Risk Evaluation and Mitigation Strategies (REMS) to help, e.g., ensure that substantially no drug remains in the drug delivery device so that the drug deliverable therefrom is not accessed by an unauthorized party. A person skilled in the art will appreciate that the drug holder may not have precisely zero drug therein but nevertheless be considered to have substantially no drug therein due to any number of factors, such as sensitivity of measurement equipment.

The device indicator can have a variety of configurations. For example, the device indicator can include an extension, e.g., a rod, shaft, a tube, etc., extending proximally from a proximal end of the drug holder 202. A first portion of the extension is a first color, and a second portion of the extension is a second, different color. FIG. 3 shows the first color visible through each of the viewing windows 224, 226. The first color is white as illustrated, although another color can be used. The first color being visible in a viewing window 224, 226 can indicate that the dose associated with the viewing window 224, 226, e.g., the first dose for the first viewing window 224 and the second dose for the second viewing window 226, has not occurred. The second color being visible in a viewing window 224, 226 can indicate that the dose associated with the viewing window 224, 226 has occurred. The extension is configured to move with the drug holder 202 such that when the drug holder 202 moves proximally relative to the body 212 during drug delivery, the color visible through a viewing window 224, 226 can change. In other embodiments, the extension can have instead of or in addition to different colors another marking configured to be visible through the viewing windows 224, 226 to indicate whether the first and second doses have been dispensed, such as symbols (e.g., stars, triangles, "X"s, a thumbs up, a check mark, etc.), numbers (e.g., "1" for the first dose and "2" for the second dose), and/or letters (e.g., "A" for the first dose and "B" for the second dose).

As shown in FIG. 4, the drug delivery device 200 is configured to releasably couple to an accessory sleeve 228. FIG. 4 illustrates the accessory sleeve 228 coupled to the drug delivery device 200. FIG. 5 illustrates the accessory sleeve 228 as a standalone unit decoupled from the drug delivery device 200. The accessory sleeve 228 is configured to facilitate delivery of two doses of the drug from the drug holder 202 and out of the opening 208, as discussed further below.

The accessory sleeve 228 is a tubular member with an inner passageway 230 extending therethrough. The accessory sleeve 228 is therefore cannulated. The accessory sleeve 228 is cylindrical in this illustrated embodiment but can have other shapes. The inner passageway 230 has a size and shape configured to receive the drug holder 202 therethrough, as shown in FIG. 4. Thus, in this illustrated embodiment in which the drug holder 202 has a cylindrical body, the inner passageway 230 has a cylindrical shape. A length 228L of the accessory sleeve 228 is less than a length 202L of the drug holder 202, shown in FIG. 6. Thus, as shown in FIG. 4, with the accessory sleeve 228 coupled to the drug delivery device 200 with the drug holder 202 positioned in the inner passageway 230, a proximal portion of the drug holder 202 extends proximal to the accessory sleeve 228, and a distal portion of the drug holder 202 extends distal to the accessory sleeve 228.

The accessory sleeve 228 can include a first mating feature, and the drug holder 202 can include a corresponding first mating feature configured to facilitate releasably coupling of the accessory sleeve 228 and the drug holder 202. The first mating features are configured to releasably mate together to help hold the drug holder 202 and the accessory sleeve 228 in an initial position relative to one another, which may help prevent premature decoupling of the accessory sleeve 228 and the drug holder 202. The initial position is shown in FIG. 4. The first mating features can have a variety of configurations. For example, the first mating feature of the accessory sleeve 228 can include a protrusion, e.g., a dimple, a detent, etc., and the first mating feature of the drug holder 202 can include a depression, e.g., a half-dome, a slot, etc., configured to releasably seat the protrusion therein. For another example, the first mating feature of the accessory sleeve 228 can include a depression, and the first mating feature of the drug holder 202 can include a protrusion configured to be releasably seated in the depression. For yet another example, the first mating features can each include a magnet. In some embodiments, the body 212 can include a first mating feature instead of or in addition to the drug holder 202 such that the first mating feature of the accessory sleeve 228 is configured to releasably mate to the first mating feature of the body 212.

In an exemplary embodiment of using the drug delivery device 200 and the accessory sleeve 228, the drug delivery device 200 has an initial configuration, shown in FIG. 2, in which the accessory sleeve 228 is not coupled to the drug delivery device 200. The first color is visible through each of the viewing windows 224, 226. The accessory sleeve 228 is then coupled to the drug delivery device 200 by positioning the drug holder 202 within the inner passageway 230, as shown in FIG. 4. In some embodiments, a user of the drug delivery device 200 couples together the accessory sleeve 228 and the drug holder 202. In other embodiments, the accessory sleeve 228 and the drug holder 202 are pre-coupled together such that the user receives the drug delivery device 200 and the accessory sleeve 228 already coupled together. The accessory sleeve 228 is positioned as proximal as possible relative to the drug holder 202 with a proximal surface of the accessory sleeve 228 abutting a distal surface of the body 212. The abutting of the distal and proximal surfaces is configured to prevent the accessory sleeve 228 from moving proximally with the drug holder 202 when the drug holder 202 is moved proximally to cause delivery of the first drug dose. The relative locations of the first mating features of the accessory sleeve 228 and the drug holder 202 (and/or the body 212) are configured to properly position the accessory sleeve 228 relative to the drug holder 202 when the first mating features are mated together.

With the accessory sleeve 228 and the drug delivery device 200 mated together, the drug holder 202 is moved proximally, e.g., pushed by a user, relative to the body 212 and the accessory sleeve 228 to cause a first dose of the drug to be delivered. The accessory sleeve 228 is configured to stop proximal movement of the drug holder 202 after the drug holder 202 has moved a distance that is defined by a length 202D (see FIG. 4) of the drug holder 202 that extends distally beyond the accessory sleeve 228. The accessory sleeve 228 prevents the drug holder 202 from moving proximally more than the distance defined by the length 202D since the distal ends of the accessory sleeve 228 and the drug holder 202 will become aligned such that the user cannot continue pushing the drug holder 202 proximally. FIG. 7 illustrates the drug delivery device 200 and the accessory sleeve 228 after the first dose has been delivered. The second color is visible through the first viewing window 224 to indicate that the first dose has been delivered. The first color remains visible through the second viewing window 226 to indicate that the second dose has not been delivered.

In an exemplary embodiment, as shown in FIG. 7, a distal end of the accessory sleeve 228 is configured to be aligned with a distal end of the body 212, e.g., with distal ends of opposed legs 236 of the handle portion 216, when the accessory sleeve 228 stops proximal movement of the drug holder 202 after the drug holder 202 has moved the distance that is defined by the length 202D. The body 212 can thus be configured to cooperate with the accessory sleeve 228 to stop the drug holder's proximal movement since the user's finger can abut the body's distal end when the distal ends of the accessory sleeve 228 and the drug holder 202 have become aligned.

In embodiments in which the drug holder 202 instead of or in addition to the body 212 includes the first mating feature, the accessory sleeve 228 can include a second mating feature, and the drug holder 202 can include a corresponding second mating feature configured to facilitate releasably coupling of the accessory sleeve 228 and the drug holder 202. The second mating features are configured to releasably mate together to help hold the drug holder 202 and the accessory sleeve 228 in an actuated position relative to one another, which may help prevent premature decoupling of the accessory sleeve 228 and the drug holder 202. The actuated position is shown in FIG. 7. The second mating features can have a variety of configurations, similar to that discussed above regarding the first mating features of the accessory sleeve 228 and the drug holder 202.

After the first dose has been delivered, e.g., after the first stage of operation, the accessory sleeve 228 is removed from the drug delivery device 200, as shown in FIG. 8. The drug holder 202 is thus free to move further proximally relative to the body 212. With the accessory sleeve 228 and the drug delivery device 200 decoupled from one another, the drug holder 202 is moved proximally, e.g., pushed by a user, relative to the body 212 to cause a second dose of the drug to be delivered. The body 212 includes a stop body 232 that includes a stop face 234 (see FIG. 3) configured to stop the proximal movement of the drug holder 202 after the drug holder 202 by abutting a proximal surface of the drug holder 202. FIG. 9 illustrates the drug delivery device 200 after the second dose has been delivered. The second color is visible through the second viewing window 226 to indicate that the second dose has been delivered. The second color remains visible through the first viewing window 224 to indicate that the first dose has been delivered.

The drug delivery device 200 in this illustrated embodiment is not powered, e.g., does not include any electrical components such as a processor, a sensor, a memory, a communications interface, etc. The drug delivery device 200 thus does not need to include a power supply though a power supply could be included, e.g., for connectivity, for powering a light source of the drug delivery device, etc.

The drug delivery device 200 can be provided as part of a kit including the drug delivery device 200 and the accessory sleeve 228. The accessory sleeve 228 can be provided without yet being coupled to the drug delivery device 200, or the accessory sleeve 228 can be provided coupled to the drug delivery device 200.

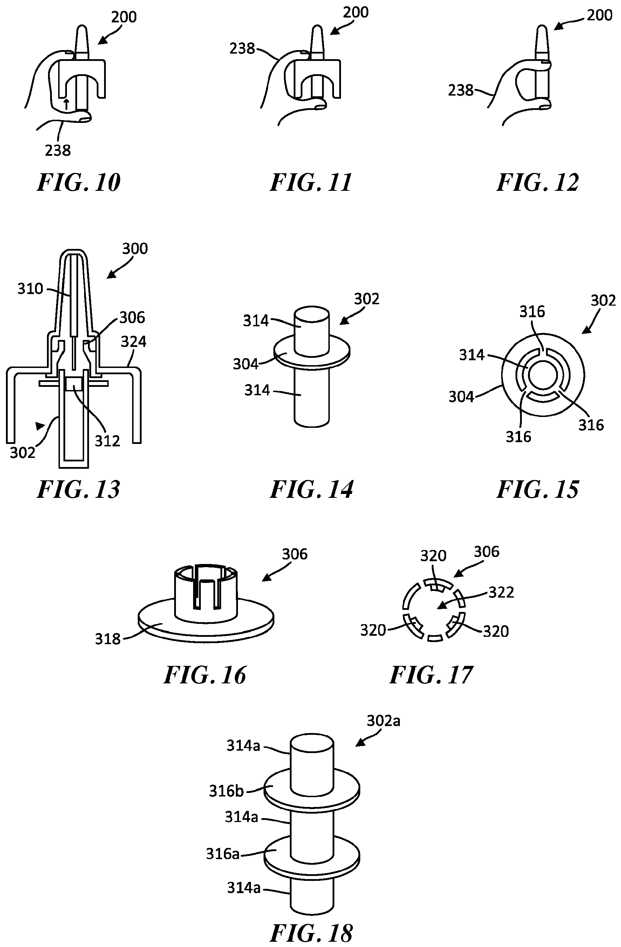

In other embodiments, the accessory sleeve 228 can be omitted, and the body 212 of the drug delivery device 200 can be configured to limit proximal movement of the drug holder 202 in the first stage of operation as discussed above, e.g., with the distal end of the body 212 limiting the drug holder's proximal movement. To allow for the second stage of operation, the user can reorient their hand relative to the body 212 and the drug holder 202 so the body's distal end no longer impedes proximal movement of the user's hand and thus the drug holder 202. FIG. 10 illustrates one embodiment of a position of a user's hand 236 relative to the drug delivery device 200 at a start of the first stage of operation. FIG. 11 illustrates the user's hand 236 moved from the position at FIG. 10 at the end of the first stage of operation with the hand abutting the body's distal end. FIG. 12 illustrates the user's hand reoriented form FIG. 11 before the start of the second stage of operation.

FIG. 13 illustrates another exemplary embodiment of the drug delivery device 100 of FIG. 1. FIG. 13 illustrates a drug delivery device 300 configured to expel a drug into a nose of a patient in two stages of operation. In a first stage of operation a first dose of the drug is delivered, and in a second stage of operation a second dose of the drug is delivered. The drug delivery device 300 is configured and used similar to the drug delivery device 200 of FIG. 2 except that the drug delivery device 300 does not include an accessory sleeve. The drug delivery device 300 in this illustrated embodiment includes a drug holder 302 including a flange 304 thereon, and the drug delivery device 300 includes a centerpiece 306 configured to interact with the drug holder 302 to facilitate delivery of two doses of the drug from the drug holder 302 and out of the drug delivery device's opening 308. FIG. 13 also illustrates a piston 310 of the drug delivery device 300 that is configured to pierce through or puncture a seal member 312 of the drug holder 202.

As shown in FIGS. 13-15, the flange 304 is attached to an elongate body 314 of the drug holder 302 via a plurality of frangible legs 316. The drug holder 302 includes three frangible legs 316 in this illustrated embodiment but can include another number, e.g., two, four, five, six, etc. The frangible legs 316 can be made from any of a variety of materials configured to allow breakage of the legs 316, such as a polymer or a metal. The legs 316 have a sufficiently small thickness to allow for their breakage. The thickness will vary based on the material of the legs 316. The flange 304, e.g., a proximal surface thereof, abuts the drug delivery device 300, e.g., an exterior distal surface of drug delivery device's body 324. **In** addition to or instead of the frangible legs 316 being made from a material to allow for breakage thereof, the frangible legs 316 can have a geometry such as a thinned region, can be chemically treated, and/or can be heat treated to facilitate breakage thereof.

As shown in FIGS. 13, 16, and 17, the centerpiece 306 includes a distal base 318 and a plurality of protrusions 320 that extend radially inward. The centerpiece 306 includes three protrusions 320 in this illustrated embodiment but can include another number, e.g., two, four, five, six, etc. A centerpiece 306 is cannulated with an inner passageway 322 extending therethrough.

**In** an exemplary embodiment of using the drug delivery device 300, the drug delivery device 300 has an initial configuration, shown in FIG. 13, in which all of the frangible legs 316 of the drug holder 302 are intact, e.g., not broken. The drug holder 302 is moved proximally, e.g., pushed by a user, relative to the drug delivery device's body 324 to cause a first dose of the drug to be delivered. The body 314 of the drug holder 302 moves proximally, but the flange 304 of the drug holder 302 does not move proximally due to the flange's abutment with the body 314 of the drug delivery device 300. Consequently, the proximal movement of the drug holder's body 314 causes the frangible legs 316 to break due to the proximal force being applied to the body 314. The centerpiece 306 is configured to stop proximal movement of the drug holder's body 314 by the centerpiece's protrusions 320, e.g., a distal surface thereof, coming into contact with a proximal end of the drug holder's body 314.

After the first dose has been delivered, e.g., after the first stage of operation, the drug holder 302 is moved further proximally relative to the drug delivery device's body 324 to cause a second dose of the drug to be delivered. The force required to move the drug holder 302 proximally in the second stage of operation is greater than the force required to move the drug holder 302 proximally in the first stage of operation. The force required to move the drug holder 302 proximally in the first stage of operation is a force sufficient to cause the frangible legs 316 to break. The force required to move the drug holder 302 proximally in the second stage of operation is a force sufficient to cause the proximal end of the drug holder 302 to pass the protrusions 320 of the centerpiece 306. The protrusions 320 can be resilient members configured to flex proximally when a sufficient force is applied thereto such that when the sufficient force (or greater) is applied to the protrusions 320 by the drug holder 302 pushing proximally thereagainst, the protrusions 320 can flex proximally to allow proximal movement of the drug holder 302. The force sufficient to cause the protrusions 320 to flex will vary based on a thickness of and on a material, e.g., a polymer or a metal, of the protrusions 320.

In other embodiments, the centerpiece 306 can be omitted, and the drug holder 302 can include a second flange in addition to the flange 304. FIG. 18 illustrates such an embodiment of a drug holder 302a that is configured and used similar to the drug holder 302 except that the drug holder 302a includes a first, distal flange 304a and a second, proximal flange 304b. Each of the flanges 304a, 304b is attached to a body 314a of the drug holder 302a with a plurality of frangible legs similar to the frangible legs 316. In an exemplary embodiment of using the drug holder 302a, the drug holder 302a is moved proximally relative to the drug delivery device's body 324 to cause a first dose of the drug to be delivered. The proximal movement of the drug holder's body 314s causes the frangible legs of the first flange 316a to break due to the proximal force being applied to the body 314a similar to that discussed above regarding the flange 314. The second flange 314a is configured to stop proximal movement of the drug holder's body 314a by the second flange's frangible legs, e.g., distal surfaces thereof, coming into contact with a proximal end of the drug holder's body 314a.

After the first dose has been delivered, e.g., after the first stage of operation, the drug holder 302a is moved further proximally relative to the drug delivery device's body 324 to cause a second dose of the drug to be delivered. The force required to move the drug holder 302 proximally in the second stage of operation is greater than the force required to move the drug holder 302 proximally in the first stage of operation. The force required to move the drug holder 302 proximally in the first stage of operation is a force sufficient to cause the frangible legs of the first flange 314a to break. The force required to move the drug holder 302 proximally in the second stage of operation is a force sufficient to cause the frangible legs of the second flange 314b to break. To provide for the force required to move the drug holder 302 proximally in the second stage of operation being greater than the force required to move the drug holder 302 proximally in the first stage of operation the frangible legs of the second flange 314b can, for example, be thicker and/or be made of a harder material than the frangible legs of the first flange 314a.

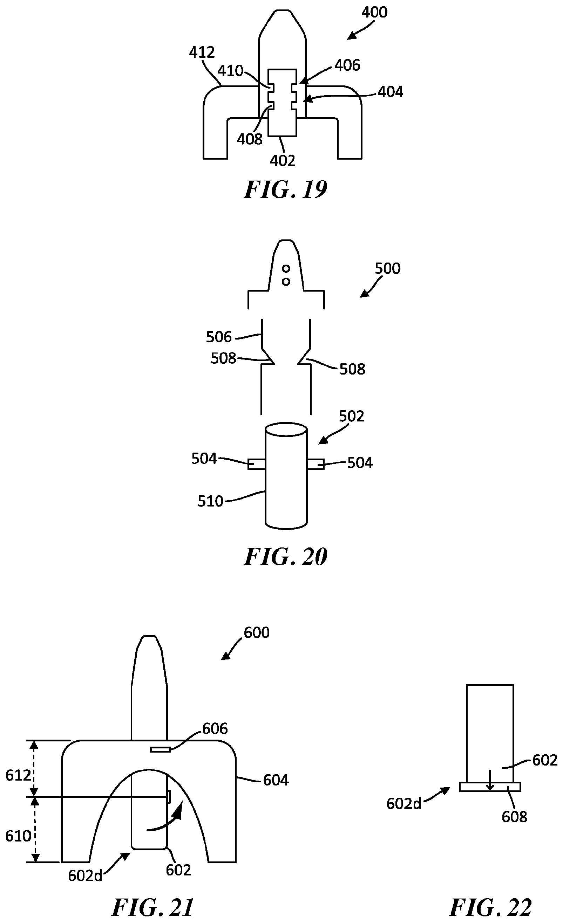

FIG. 19 illustrates another exemplary embodiment of the drug delivery device 100 of FIG. 1. FIG. 19 illustrates a drug delivery device 400 configured to expel a drug into a nose of a patient in two stages of operation. In a first stage of operation a first dose of the drug is delivered, and in a second stage of operation a second dose of the drug is delivered. The drug delivery device 400 is configured and used similar to the drug delivery device 300 of FIG. 13 except that the centerpiece 306 is absent, and a drug holder 402 of the drug delivery device 400 does not include first and second flanges 314a, 314b. Instead, the drug holder 402 includes first and second grooves 404, 406, and the drug delivery device's body 412 includes first and second protrusions 408, 410 that extend radially inward and are configured to variously engage the first and second grooves 404, 406. The first and second grooves 404, 406 extend circumferentially around the drug holder 402 in this illustrated embodiment, which may facilitate coupling of the drug holder 402 to the drug delivery device's body 412 since the drug holder 402 can be coupled to the body 412 at any rotational position of the drug holder 402. However, one or both of the grooves 404, 406 can extend around only a partial circumference of the drug holder 402.

In an exemplary embodiment of using the drug delivery device 400, the drug delivery device 400 has an initial configuration, shown in FIG. 19, in which the first protrusion 408 is seated in the first groove 404, and the second protrusion 410 is seated in the second groove 406. The drug holder 402 is moved proximally, e.g., pushed by a user, relative to the drug delivery device's body 412 to cause a first dose of the drug to be delivered. The proximal movement of the drug holder 402 causes the first protrusion 408 to become unseated from the first groove 404, the second protrusion 410 to become unseated from the second groove 406, and the second protrusion 410 to become seated in the first groove 404. The seating of the second protrusion 410 in the first groove 404 is configured to stop proximal movement of the drug holder 402.

After the first dose has been delivered, e.g., after the first stage of operation, the drug holder 402 is moved further proximally relative to the drug delivery device's body 412 to cause a second dose of the drug to be delivered. The further proximal movement of the drug holder 402 causes the second protrusion 410 to become unseated from the first groove 404.

The protrusions 408, 410 can be resilient members configured to flex proximally when a sufficient force is applied thereto such that when the sufficient force (or greater) is applied to the protrusions 408, 410 by the drug holder 402 pushing proximally thereagainst, the protrusions 408, 410 can flex proximally to allow proximal movement of the drug holder 402. The force sufficient to cause the protrusions 408, 410 to flex will vary based on a thickness of and on a material, e.g., a polymer or a metal, of the protrusions 408, 410.

FIG. 20 illustrates another exemplary embodiment of the drug delivery device 100 of FIG. 1. FIG. 20 illustrates a drug delivery device 500 configured to expel a drug into a nose of a patient in two stages of operation. In a first stage of operation a first dose of the drug is delivered, and in a second stage of operation a second dose of the drug is delivered. The drug delivery device 500 is configured and used similar to the drug delivery device 300 of FIG. 13 except that the centerpiece 306 is absent. Also, the drug delivery device 500 of FIG. 15 includes a drug holder 502 that includes a plurality of frangible tabs 504, and the drug delivery device's body 506 includes a plurality of grooves 508 each configured to seat one of the tabs 504 therein. The drug holder 502 includes two tabs 504 in this illustrated embodiment but can include another number, e.g., one, three, four, five, etc. The body 506 includes two grooves 508 in this illustrated embodiment but can include another number, e.g., one, three, four, five, etc.

In an exemplary embodiment of using the drug delivery device 500, the drug delivery device 500 has an initial configuration in which the frangible tabs 504, e.g., proximal surfaces thereof, abut the drug delivery device 500, e.g., an exterior distal surface of the body 506. The drug holder 502 is moved proximally, e.g., pushed by a user, relative to the drug delivery device's body 506 to cause a first dose of the drug to be delivered. A body 510 of the drug holder 302 moves proximally with the frangible tabs 504 of the drug holder 502. The proximal movement of the drug holder 502 causes the frangible tabs 504 to flex, to enter the body 506, and to become seated in the grooves 508. The seating of the tabs 504 in the grooves 508 is configured to stop proximal movement of the drug holder 502.

After the first dose has been delivered, e.g., after the first stage of operation, the drug holder 502 is moved further proximally relative to the drug delivery device's body 506 to cause a second dose of the drug to be delivered. The further proximal movement of the drug holder 502 causes the frangible tabs 504 to break. The force required to move the drug holder 502 proximally in the second stage of operation is greater than the force required to move the drug holder 502 proximally in the first stage of operation. The force required to move the drug holder 502 proximally in the first stage of operation is a force sufficient to cause the frangible tabs 504 to flex but not break. The force required to move the drug holder 502 proximally in the second stage of operation is a force sufficient to cause the frangible tabs 504 to break. The forces will vary based on a thickness of and on a material, e.g., a polymer or a metal, of the frangible tabs 504 and the body 506.

FIG. 21 illustrates another exemplary embodiment of the drug delivery device 100 of FIG. 1. FIG. 21 illustrates a drug delivery device 600 configured to expel a drug into a nose of a patient in two stages of operation. **In** a first stage of operation a first dose of the drug is delivered, and in a second stage of operation a second dose of the drug is delivered. The drug delivery device 600 is configured and used similar to the drug delivery device 200 of FIG. 2 except that instead of the drug delivery device's drug holder 602 translating axially relative to the drug delivery device's body 604 to cause drug delivery, the drug holder 602 is configured to rotate relative to the drug delivery device's body 604 to cause drug delivery. The body 604 includes a cam 606 that extends radially inward from an interior surface thereof and that is configured to guide the rotation of the drug holder 602 in response to axial pushing on a distal end 602d of the drug holder 602. The drug holder 602 includes a helical groove formed in an exterior surface thereof in which the cam 606 is configured to slide. Thus, in response to a proximal force being applied to the distal end 602d of the drug holder 602, the drug holder 602 rotates. As shown in FIG. 22, the distal end 602d of the drug holder 602 can include a pad 608 thereon. The pad 408 is configured to provide friction with a user's thumb or finger that is applying the proximal force to the drug holder's distal end 602d. The friction may help encourage the drug holder's rotational movement in response to an axial force being applied to the drug holder 602.

**In** the first stage of operation, the drug holder 602 is configured to rotate a first amount such that the drug holder 602 moves a first distance 610 proximally relative to the body 604. In the first stage of operation, the drug holder 602 is configured to rotate a second amount such that the drug holder 602 moves a second distance 612 proximally relative to the body 604. The helical groove of the drug holder 602 can include a friction member along its length that is configured to cooperate with the cam 606 to stop the rotational movement of the drug holder 602 to end the first stage of operation. The friction member can have a variety of configurations. For example, the friction member can include a depression configured to seat the cam 606 therein. A force applied to the drug holder 602 that is greater than a force to move the drug holder 602 in the first stage of operation can be configured to move the cam 606 out of the depression to allow for occurrence of the second stage of operation.

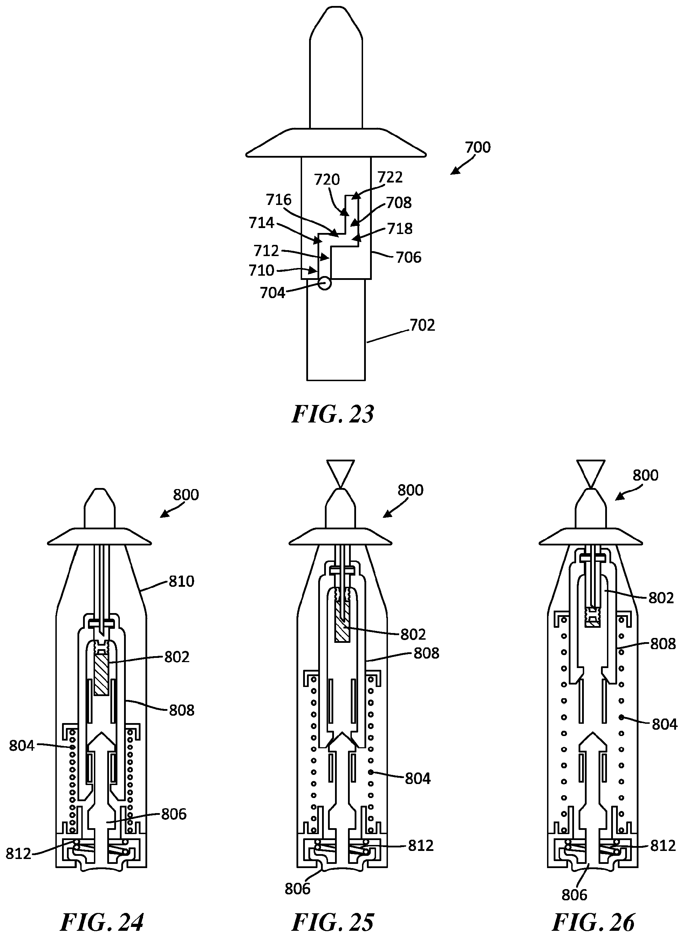

FIG. 23 illustrates another exemplary embodiment of the drug delivery device 100 of FIG. 1. FIG. 23 illustrates a drug delivery device 700 configured to expel a drug into a nose of a patient in two stages of operation. **In** a first stage of operation a first dose of the drug is delivered, and in a second stage of operation a second dose of the drug is delivered. The drug delivery device 700 is configured and used similar to the drug delivery device 600 of FIG. 21 except that a drug holder 702 of the drug delivery device 700 includes a cam 704 and a body 706 of the drug delivery device 700 includes a track 708 in which the cam 704 is slidably movable. Also, in this illustrated embodiment, the drug holder 702 being pushed in a proximal direction causes the drug holder 702 to move axially, not rotationally as in the embodiment of FIG. 21.

In an exemplary embodiment of using the drug delivery device 700, the drug delivery device 700 has an initial configuration, shown in FIG. 23, in which the cam 704 is positioned just distal to an open distal end 710 of the track 708. The drug holder 702 is moved proximally, e.g., pushed by a user, relative to the drug delivery device's body 706 to cause a first dose of the drug to be delivered. The cam 704 enters into and slides in a first longitudinal portion 712 of the track 708 during the first stage of operation. A proximal end 714 of the first longitudinal or vertical portion 712 of the track 708 is configured to stop the proximal movement of the drug holder 702 by the cam 704 abutting the proximal end 714 of the first longitudinal portion 712 of the track 708. In other embodiments, the distal end of the track 708 is closed instead of being open, and with the drug delivery device 700 in the initial configuration the cam 704 is positioned within the track 708 at the track's closed distal end.

After the first dose has been delivered, e.g., after the first stage of operation, the drug holder 702 is rotated relative to the body 706. The rotation of the drug holder 702 causes the cam 704 to slide within an axial or horizontal portion 716 of the track 708. The drug holder 702 cannot be rotated relative to the body 706 during the first stage of operation due to the cam's location within the first longitudinal portion 712 of the track 708. In embodiments in which, in the initial configuration, the cam 704 is positioned within the track 708 at the track's closed distal end, the drug holder 702 is prevented from rotating relative to the body 706 before the first stage of operation due to the cam's location within the first longitudinal portion 712 of the track 708. An end 718 of the axial portion 716 of the track 708 is configured to stop the rotational movement of the drug holder 702 by the cam 704 abutting the end 718.

After the first dose has been delivered, e.g., after the first stage of operation, the drug holder 702 is moved further proximally relative to the drug delivery device's body 706 to cause a second dose of the drug to be delivered. The cam 704 slides in a second longitudinal or vertical portion 720 of the track 708 during the second stage of operation. A proximal end 722 of the second longitudinal portion 720 of the track 708 is configured to stop the proximal movement of the drug holder 702 by the cam 704 abutting the proximal end 722 of the second longitudinal portion 720 of the track 708.

FIG. 24 illustrates another exemplary embodiment of the drug delivery device 100 of FIG. 1. FIG. 24 illustrates a drug delivery device 800 configured to expel a drug into a nose of a patient in two stages of operation. In a first stage of operation a first dose of the drug is delivered, and in a second stage of operation a second dose of the drug is delivered. The drug delivery device 800 is configured and used similar to the drug delivery device 300 of FIG. 13 except that the drug delivery device 800 in this illustrated embodiment is spring-driven. The drug delivery device 800 includes a push spring 804 configured to drive movement of a drug holder 802 of the drug delivery device 800 in response to actuation of the drug delivery device's actuator 806, which is a button in this illustrated embodiment. The drug delivery device 800 also includes an internal sleeve 808 that is internal to the drug delivery device's body 810 configured to cooperate with the actuator 806 in the first and second stages of operation.

In an exemplary embodiment of using the drug delivery device 800, the drug delivery device 800 has an initial configuration, shown in FIG. 24, in which the push spring 804 is in a compressed configuration and the actuator 806 is operatively engaged with the internal sleeve 808. The actuator 806 is actuated, e.g., by being pushed on a distal surface thereof by a user, which causes the actuator 806 to release the push spring 804. The release of the push spring 804 allows the push spring 804 to move from the compressed configuration to an expanded configuration, to which the push spring 804 is biased. The push spring 804 expands proximally. The proximal expansion of the push spring 804 causes the internal sleeve 808 to move proximally. The drug holder 802 is fixed to the internal sleeve 808, so the drug holder 802 also moves proximally to cause a first dose of the drug to be delivered, as shown in FIG. 25. The drug delivery device 800 includes a return spring 812 operatively engaged with the actuator 806 that causes the actuator 806 to return to its initial position after the first dose of the drug has been delivered.

After the first dose has been delivered, e.g., after the first stage of operation, the actuator 806 is actuated again, which repeats the above-discussed process in which the push spring 804 causes the internal sleeve 808 and the drug holder 802 to move proximally such that drug is expelled out of the drug delivery device 800, as shown in FIG. 26.

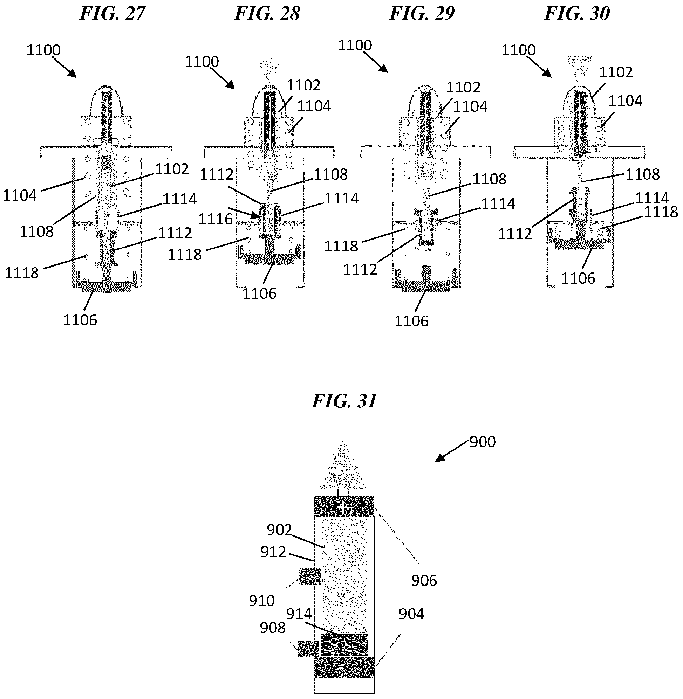

FIG. 27 illustrates another exemplary embodiment of the drug delivery device 100 of FIG. 1. FIG. 27 illustrates a drug delivery device 1100 configured to expel a drug into a nose of a patient in two stages of operation. In a first stage of operation a first dose of the drug is delivered, and in a second stage of operation a second dose of the drug is delivered. The drug delivery device 1100 is configured and used similar to the drug delivery device 800 of FIG. 24 except that the drug delivery device 1100 in this illustrated embodiment includes a rotation mechanism 1114. Similar to the drug delivery device 800 of FIG. 24, the drug delivery device 1100 includes a push spring 1104 configured to drive movement of a drug holder 1102 of the drug delivery device 1100 in response to actuation of the drug delivery device's actuator 1106, which includes a button in this illustrated embodiment. The drug delivery device 1100 also includes an internal sleeve 1108 that is internal to the drug delivery device's body 1110 configured to cooperate with the actuator 1106 in the first and second stages of operation.

In an exemplary embodiment of using the drug delivery device 1100, the drug delivery device 1100 has an initial configuration, shown in FIG. 27, in which the push spring 1104 is in an expanded configuration, the actuator 1106 is operatively engaged with the internal sleeve 1108 via a coupling member 1112, and the coupling member 1112 is positioned distal to the rotation mechanism 1114. The actuator 1106 is actuated, e.g., by being pushed on a distal surface thereof by a user, which causes the drug delivery device's return spring 1118 to compress, which causes the coupling member 1112 to move proximally to be positioned within a collar 1116 defined by the rotation mechanism 1114, and which causes the sleeve 1108 to move proximally. The proximal movement of the actuator 1106 causes the drug holder 1102 seated therein to move proximally and thereby cause a first dose of the drug to be delivered, as shown in FIG. 28. The coupling member 1112, e.g., a proximal surface of a distal flange thereof, abutting the rotation mechanism 1114, e.g., a distal surface of the collar 1116, acts as a stop that stops proximal movement of the actuator 1106.

In response to the proximal force applied by the actuator 1106 being removed, e.g., the user stops pushing proximally on the actuator 1106, the return spring 1118 operatively engaged with the actuator 1106 causes the actuator 1106 to return to its initial position after the first dose of the drug has been delivered, as shown in FIG. 29. The distal movement of the actuator 1106 as the actuator 1106 returns to its initial position releases a proximal force being applied to the coupling member 1112 by the actuator 1106. The rotation mechanism 1114 is thus free to cause the coupling member 1112 to rotate within the collar 1118 similar to a ballpoint pen rotaton mechanism, as also shown in FIG. 29.

After the first dose has been delivered, e.g., after the first stage of operation, the actuator 1106 is actuated again, as shown in FIG. 30, which causes the return spring 1118 to compress again and the actuator 1106 to come into contact again with the coupling member 1112, which causes the coupling member 1112 to move proximally out of the collar 1116, and which causes the sleeve 1108 to move proximally. The proximal movement of the actuator 1106 causes the drug holder 1102 seated therein to move proximally and thereby cause a second dose of the drug to be delivered, as also shown in FIG. 30.

FIG. 31 illustrates another exemplary embodiment of the drug delivery device 100 of FIG. 1. FIG. 31 illustrates a drug delivery device 900 configured to expel a drug into a nose of a patient in two stages of operation. In a first stage of operation a first dose of the drug is delivered, and in a second stage of operation a second dose of the drug is delivered. The drug delivery device 900 is configured and used similar to the drug delivery device 200 of FIG. 2 except that the drug delivery device 900 uses magnet power in driving drug delivery in the first and second stages of operation. Magnet strength does not reduce with time and is stable toward environmental conditions such as temperature, humidity, etc., which may allow the drug delivery device 900 to remain actuatable over a long period of time and regardless of the environmental conditions experienced by the drug delivery device 900.

The drug delivery device 900 includes a first, distal magnet 904 located at a distal end of the drug delivery device's drug holder 902, and a second, proximal magnet 906 located at a proximal end of the drug holder 902. The magnets 904, 906 have opposite polarities, with the distal magnet 904 having a negative polarity and the proximal magnet 906 have a positive polarity, although the polarities can be reversed. The drug delivery device 900 also includes a first, distal actuator 908 and a second, proximal actuator 910. The first and second actuators 908, 910 are configured to be actuated, as discussed above, and to act as mechanical stops for drug delivery. A plume geometry of the drug expelled from the drug delivery device 900 is not dependent on a strength of a force applied to either of the actuators 908, 910 since the magnets 904, 906 are configured to provide actuation speed. A strength of the magnets 904, 906 can thus define the actuation speed and corresponding plume geometry. A user actuating the actuators 908, 910 does not push a plunger or drug holder to cause actuation, so a movement distance or stroke length of the plunger or drug holder is not noticed by the user.

In an exemplary embodiment of using the drug delivery device 900, the drug delivery device 900 has an initial configuration, shown in FIG. 31, in which the first actuator 908 acts as a mechanical stop for the first magnet 908, e.g., with a distal surface of the first actuator 908 abutting a proximal surface of the first magnet 908. The first magnet 908, which is slidably coupled to the drug delivery device's body 912, is thus prevented from being drawn proximally toward the second magnet 910, which is fixed relative to the drug delivery device's body 912. The first actuator 908 is actuated, e.g., by being pulled on by a user, which causes the first actuator 908 to release the first magnet 904, thereby allowing the first magnet 904 to move proximally toward the second magnet 906. A stopper 914 in the drug holder 902 that is operatively engaged by the first magnet 904 thus also moves proximally, by being pushed by the first magnet 904, to cause drug in the drug holder 902 to exit the drug delivery device 900. The second actuator 910 acts as a stop that stops the proximal movement of the first magnet 904, and hence the proximal movement of the stopper 914, e.g., by a distal surface of the second actuator 910 abutting the proximal surface of the first magnet 904.

After the first dose has been delivered, e.g., after the first stage of operation, the second actuator 910 is actuated, , e.g., by being pulled on by a user, which causes the second actuator 910 to release the first magnet 904, thereby allowing the first magnet 904 to again move proximally toward the second magnet 910. The stopper 914 thus also again moves proximally to cause drug in the drug holder 902 to again exit the drug delivery device 900. The second magnet 906 acts as a stop that stops the proximal movement of the first magnet 904, and hence the proximal movement of the stopper 914, e.g., by the distal surface of the second actuator 910 abutting a proximal surface of the stopper 914.

In the illustrated embodiments of the drug delivery devices 200, 300, 400, 500, 600, 700, 800, 900 of FIGS. 2, 13, 19-21, 23, 24, 27, and 31 the drug holder 202, 302, 402, 502, 602, 702, 802, 1102, 902 contains the drug therein in a single chamber for delivery in each of the first and second stages of operation. In other embodiments, the drug holder 202, 302, 402, 502, 602, 702, 802, 1102, 902 can contain the drug therein in a first chamber for delivery in the first stage of operation and in a second, separate chamber for delivery in the second stage of operation. The drugs in the first and second chambers can be the same drug as each other or can be a different drug from one another. The drug holder having two drug chambers may help ensure that a predictable, substantially equal amount of drug is delivered in each of the two stages of operation and/or may help provide a stop to end the first stage of operation. A person skilled in the art will appreciate that the drug amounts may not be precisely equal but nevertheless be considered to be substantially equal due to any number of factors, such as sensitivity of measurement equipment.

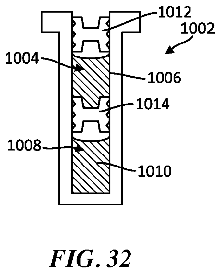

FIG. 32 illustrates one embodiment of a drug holder 1002 that includes a first chamber 1004 containing a first drug 1006 therein for delivery in the first stage of operation and a second chamber 1008 containing a second drug 1010 therein for delivery in the second stage of operation. As mentioned above, the drugs 1006, 1010 can be the same as each other or different from one another. The drug holder 1002 also includes a first seal member 1012 and a second seal member 1014. The first and second seal members 1012, 1014 are configured to be pierced or punctured as discussed above. The first seal member 1012 is proximal to both of the first and second chambers 1004, 1008. The second seal member 1014 is located between the first and second chambers 1004, 1008, e.g., is distal to the first chamber 1004 and is proximal to the second chamber 1008, such that the first and second chambers 1004, 1008 are isolated from each other prior to piercing or puncturing of the second seal member 1014. The second seal member 1014 is configured to facilitate stopping the first stage of operation, in cooperation with the other stop discussed above for the various embodiments, by providing a surface against which the piston or other piercing element abuts at the end of the first stage of operation.

Embodiments of nasal drug delivery devices disclosed herein can be designed to be disposed of after a single use, or they can be designed to be used multiple times. In either case, in at least some embodiments, the drug delivery device can be reconditioned for reuse after at least one use. Reconditioning can include any combination of the steps of disassembly of the drug delivery device, followed by cleaning or replacement of particular pieces and subsequent reassembly. In particular, the drug delivery device can be disassembled, and any number of the particular pieces or parts of the drug delivery device can be selectively replaced or removed in any combination. Upon cleaning and/or replacement of particular parts, the drug delivery device can be reassembled for subsequent use either at a reconditioning facility, or by a health care provider immediately prior to use. A person skilled in the art will appreciate that reconditioning of a drug delivery device can utilize a variety of techniques for disassembly, cleaning/replacement, and reassembly. Use of such techniques, and the resulting reconditioned drug delivery device, are all within the scope of the present application.

The present disclosure has been described above by way of example only within the context of the overall disclosure provided herein. It will be appreciated that modifications within the scope of the claims may be made without departing from the overall scope of the present disclosure.

## Claims

1. A drug delivery system (100, 200), comprising:
a dispensing head (110, 204) including a tip (112, 206) configured to be positioned in a nose of a patient, the tip having an opening (108, 208) therein;
a drug holder (102, 202) containing a drug therein;
a first stop member (228); and
a second stop member (232); **characterised in that**:
the drug holder is configured to move a first amount relative to the dispensing head in a first stage of operation in which the drug is delivered from the drug holder and out of the opening;
the drug holder is configured to move a second amount relative to the dispensing head in a second stage of operation in which the drug is delivered from the drug holder and out of the opening;
the first stop member is configured to mechanically stop movement of the drug holder relative to the dispensing head between the first and second stages of operation; and
the second stop member is configured to mechanically stop movement of the drug holder relative to the dispensing head after the second stage of operation.

2. The system (200) of claim 1, further comprising an accessory (228) that includes the first stop member, that is configured to be releasably coupled to the drug holder during the first stage of operation, and that is configured to be removed from the drug holder between the first and second stages of operation, wherein optionally the accessory has an inner passageway extending therethrough and the accessory is configured to be releasably coupled to the drug holder during the first stage of operation with the drug holder extending through the inner passageway.

3. The system (200) of claim 2, further comprising a body (212) that includes the second stop member; wherein:
the second stop member comprises an exterior surface of the body;
the drug holder is configured to move the first amount relative to the body in the first stage of operation; and
the drug holder is configured to move the second amount relative to the body in the second stage of operation.

4. The system (200) of claim **1,** further comprising a body (212) that includes the first and second stop members; wherein:
the first stop member comprises a first exterior surface of the body;
the second stop member comprises a second exterior surface of the body that is proximal to the first exterior surface;
the drug holder is configured to move the first amount proximally relative to the body in the first stage of operation; and
the drug holder is configured to move the second amount proximally relative to the body in the second stage of operation.

5. The system (300) of claim 1, wherein the drug holder (302, 302a) includes an elongate body (314, 314a) with a flange (304, 304a) extending radially outward from the elongate body, the flange being releasably attached to the elongate body with a plurality of frangible legs (316), wherein optionally the plurality of frangible legs are each configured to break during the first stage of operation.

6. The system (300) of claim 5, further comprising a centerpiece (306) that includes the first stop member; and
a body that includes the second stop member;
wherein the first stop member comprises a protrusion (320) configured to engage the flange; and
wherein the second stop member comprises an interior surface of the body configured to engage a surface of the drug holder.

7. The system (300) of claim 5, further comprising a body that includes the second stop member; wherein:
the drug holder (302a) includes a second flange (304b) extending radially outward from the elongate body (314a), the second flange being releasably attached to the elongate body with a second plurality of frangible legs that are each configured to break during the second stage of operation.

8. The system (400) of claim 1, wherein the drug holder (402) has first (404) and second (406) grooves formed therein;
the drug delivery system includes a body (412) having first (408) and second (410) protrusions extending radially inward therefrom; and
the first stop member comprises the first groove and the first protrusion, and the second stop member comprises the second groove and the second protrusion.

9. The system (500) of claim 1, wherein the drug holder (502) includes a frangible tab (504) extending radially outward therefrom; and
the drug delivery system includes a body (506) including a protrusion (508) extending radially inward therefrom that defines the first stop member, and the body includes an interior surface that defines the second stop member.

10. The system (600) of claim 1, wherein the drug holder (602) is configured to be pushed in an axial direction toward the dispensing head in each of the first and second stages of operation;
the drug holder has a helical groove formed therein; and
the drug delivery system further comprises a cam (606) configured to slide within the helical groove in response to the pushing of the drug holder, thereby causing the drug holder to rotate relative to the dispensing head.

11. The system (700) of claim 1, wherein the drug holder (702) is configured to be pushed in a longitudinal direction toward the dispensing head in each of the first and second stages of operation;
the drug holder has a protrusion (704) extending therefrom;
the drug delivery system further comprises a body (706) having a track (708) formed therein;
the protrusion is configured to slide longitudinally within the track in response to the pushing of the drug holder, thereby causing the protrusion of the drug to slide axially in the track;
the drug holder is configured to be rotated relative to the dispensing head between the first and second stages of operation; and
the protrusion is configured to slide axially within the track in response to the rotation of the drug holder.

12. The system (800, 1100) of claim 1, further comprising a body (810, 1110) with a sleeve (808, 1108) disposed therein that has the drug holder (802, 1102) fixed thereto;
an actuator (806, 1106) configured to be actuated to cause the drug to be delivered out of the opening; and
a push spring (804, 1104) configured to push the sleeve and the drug holder proximally in response to the actuation of the actuator;
wherein the first stop member includes a first surface of the sleeve; and
wherein the second stop member includes a second surface of the sleeve.

13. The system (900) of claim 1, further comprising a first actuator (908) that includes the first stop member and that is configured to be actuated to cause the drug to be delivered out of the opening in the first stage of operation;
a second actuator (910) that includes the second stop member and that is configured to be actuated to cause the drug to be delivered out of the opening in the first stage of operation;
a proximal magnet (906); and
a distal magnet (904) configured to be drawn proximally toward the proximal magnet in response to the actuation of the first actuator and in response to the actuation of the second actuator.

14. The system of any one of the preceding claims, further comprising a viewing window (224, 226) configured to allow a user to visualize whether or not each of the first and second stages of operation have occurred.

15. The system of any one of the preceding claims, wherein the drug is one of ketamine, esketamine, naloxone, and sumatriptan.

## Patentansprüche

1. Arzneimittelabgabesystem (100, 200), umfassend:
einen Abgabekopf (110, 204), der eine Spitze (112, 206) einschließt, die konfiguriert ist, um in einer Nase eines Patienten positioniert zu werden, wobei die Spitze eine Öffnung (108, 208) darin aufweist;
einen Arzneimittelbehälter (102, 202), der ein Arzneimittel darin enthält;
ein erstes Anschlagelement (228); und
ein zweites Anschlagelement (232); **dadurch gekennzeichnet, dass:**
der Arzneimittelbehälter konfiguriert ist, um sich in einer ersten Betriebsphase, in der das Arzneimittel aus dem Arzneimittelbehälter und aus der Öffnung abgegeben wird, um einen ersten Betrag relativ zum Abgabekopf zu bewegen;
der Arzneimittelbehälter konfiguriert ist, um sich in einer zweiten Betriebsphase, in der das Arzneimittel aus dem Arzneimittelbehälter und aus der Öffnung abgegeben wird, um einen zweiten Betrag relativ zum Abgabekopf zu bewegen;
das erste Anschlagelement konfiguriert ist, um die Bewegung des Arzneimittelbehälters relativ zum Abgabekopf zwischen der ersten und zweiten Betriebsphase mechanisch zu stoppen; und
das zweite Anschlagelement konfiguriert ist, um die Bewegung des Arzneimittelbehälters relativ zum Abgabekopf nach der zweiten Betriebsphase mechanisch zu stoppen.

2. System (200) nach Anspruch 1, ferner umfassend ein Zubehörteil (228), das das erste Anschlagelement einschließt, das konfiguriert ist, um während der ersten Betriebsphase lösbar mit dem Arzneimittelbehälter gekoppelt zu sein, und das konfiguriert ist, um zwischen der ersten und zweiten Betriebsphase vom Arzneimittelbehälter entfernt zu werden, wobei das Zubehörteil optional einen inneren Durchgang aufweist, der sich durch dieses hindurch erstreckt, und das Zubehörteil konfiguriert ist, um während der ersten Betriebsphase lösbar mit dem Arzneimittelbehälter gekoppelt zu sein, wobei sich der Arzneimittelbehälter durch den inneren Durchgang erstreckt.

3. System (200) nach Anspruch 2, ferner umfassend einen Körper (212), der das zweite Anschlagelement einschließt; wobei:
das zweite Anschlagelement eine Außenfläche des Körpers umfasst;
der Arzneimittelbehälter konfiguriert ist, um in der ersten Betriebsphase den ersten Betrag relativ zum Körper zu bewegen; und
der Arzneimittelbehälter konfiguriert ist, um in der zweiten Betriebsphase den zweiten Betrag relativ zum Körper zu bewegen.

4. System (200) nach Anspruch 1, ferner umfassend einen Körper (212), der das erste und das zweite Anschlagelement einschließt; wobei:
das erste Anschlagelement eine erste Außenfläche des Körpers umfasst;
das zweite Anschlagelement eine zweite Außenfläche des Körpers umfasst, die proximal zur ersten Außenfläche liegt;
der Arzneimittelbehälter konfiguriert ist, um in der ersten Betriebsphase den ersten Betrag proximal relativ zum Körper zu bewegen; und
der Arzneimittelbehälter konfiguriert ist, um in der zweiten Betriebsphase den zweiten Betrag proximal relativ zum Körper zu bewegen.

5. System (300) nach Anspruch 1, wobei der Arzneimittelbehälter (302, 302a) einen länglichen Körper (314, 314a) mit einem Flansch (304, 304a) einschließt, der sich radial von dem länglichen Körper nach außen erstreckt, wobei der Flansch mit einer Vielzahl von zerbrechlichen Schenkeln (316) lösbar an dem länglichen Körper befestigt ist, wobei die Vielzahl der zerbrechlichen Schenkel optional jeweils konfiguriert ist, um während der ersten Betriebsphase zu brechen.

6. System (300) nach Anspruch 5, ferner umfassend ein Mittelstück (306), das das erste Anschlagelement einschließt; und
einen Körper, der das zweite Anschlagelement einschließt;
wobei das erste Anschlagelement einen Vorsprung (320) umfasst, der konfiguriert ist, um mit dem Flansch einzugreifen; und
wobei das zweite Anschlagelement eine Innenfläche des Körpers umfasst, die konfiguriert ist, um mit einer Oberfläche des Arzneimittelbehälters in Eingriff zu kommen.

7. System (300) nach Anspruch 5, ferner umfassend einen Körper, der das zweite Anschlagelement einschließt; wobei:
der Arzneimittelbehälter (302a) einen zweiten Flansch (304b) einschließt, der sich radial vom länglichen Körper (314a) nach außen erstreckt, wobei der zweite Flansch mit einer zweiten Vielzahl von zerbrechlichen Schenkeln, die jeweils so konfiguriert sind, dass sie während der zweiten Betriebsphase brechen, lösbar am länglichen Körper befestigt ist.

8. System (400) nach Anspruch 1, wobei in dem Arzneimittelbehälter (402) eine erste (404) und eine zweite (406) Nut ausgebildet sind;
das Arzneimittelabgabesystem einen Körper (412) mit einem ersten (408) und einem zweiten (410) Vorsprung, die sich radial davon nach innen erstrecken, einschließt; und
das erste Anschlagelement umfasst die erste Nut und den ersten Vorsprung und das zweite Anschlagelement umfasst die zweite Nut und den zweiten Vorsprung.

9. System (500) nach Anspruch 1, wobei der Arzneimittelbehälter (502) eine zerbrechliche Lasche (504) einschließt, die sich radial von diesem nach außen erstreckt; und
das Arzneimittelabgabesystem einen Körper (506) einschließlich eines Vorsprungs (508) einschließt, der sich radial von diesem nach innen erstreckt und das erste Anschlagelement definiert, und der Körper eine Innenfläche einschließt, die das zweite Anschlagelement definiert.

10. System (600) nach Anspruch 1, wobei der Arzneimittelbehälter (602) konfiguriert ist, um in der ersten und zweiten Betriebsphase jeweils in eine axiale Richtung in Richtung des Abgabekopfes gedrückt zu werden;
der Arzneimittelbehälter eine darin ausgebildete spiralförmige Nut aufweist; und
das Arzneimittelabgabesystem ferner eine Nocke (606) umfasst, die konfiguriert ist, um als Reaktion auf das Drücken des Arzneimittelbehälters in der spiralförmigen Nut zu gleiten, wodurch der Arzneimittelbehälter veranlasst wird, sich relativ zum Abgabekopf zu drehen.

11. System (700) nach Anspruch 1, wobei der Arzneimittelbehälter (702) konfiguriert ist, um in der ersten und zweiten Betriebsphase jeweils in eine Längsrichtung in Richtung des Abgabekopfes gedrückt zu werden;
der Arzneimittelbehälter einen Vorsprung (704) aufweist, der sich von diesem erstreckt;
das Arzneimittelabgabesystem ferner einen Körper (706) mit einer darin ausgebildeten Schiene (708) umfasst;
der Vorsprung konfiguriert ist, um als Reaktion auf das Drücken des Arzneimittelbehälters in Längsrichtung innerhalb der Schiene zu gleiten, wodurch der Vorsprung des Arzneimittels veranlasst wird, axial in der Schiene zu gleiten;
der Arzneimittelbehälter konfiguriert, um zwischen der ersten und zweiten Betriebsphase relativ zum Abgabekopf gedreht zu werden; und
der Vorsprung konfiguriert ist, um als Reaktion auf die Drehung des Arzneimittelbehälters axial innerhalb der Schiene zu gleiten.

12. System (800, 1100) nach Anspruch 1, ferner umfassend einen Körper (810, 1110) mit einer darin angeordneten Hülse (808, 1108), an der der Arzneimittelbehälter (802, 1102) befestigt ist;
einen Aktuator (806, 1106), der konfiguriert ist, um betätigt zu werden, um die Abgabe des Arzneimittels aus der Öffnung zu bewirken; und
eine Druckfeder (804, 1104), die konfiguriert ist, um die Hülse und den Arzneimittelbehälter als Reaktion auf die Betätigung des Aktuators proximal zu drücken;
wobei das erste Anschlagelement eine erste Oberfläche der Hülse einschließt; und wobei das zweite Anschlagelement eine zweite Oberfläche der Hülse einschließt.

13. System (900) nach Anspruch 1, ferner umfassend einen ersten Aktuator (908), der das erste Anschlagelement einschließt und konfiguriert ist, um betätigt zu werden, um zu veranlassen, dass in der ersten Betriebsphase das Arzneimittel aus der Öffnung abgegeben wird;
einen zweiten Aktuator (910), der das zweite Anschlagelement einschließt und konfiguriert ist, um betätigt zu werden, um zu veranlassen, dass in der ersten Betriebsphase das Arzneimittel aus der Öffnung abgegeben wird;
einen proximalen Magneten (906); und
einen distalen Magneten (904), der konfiguriert ist, um als Reaktion auf die Betätigung des ersten Aktuators und als Reaktion auf die Betätigung des zweiten Aktuators proximal in Richtung des proximalen Magneten gezogen zu werden.

14. System nach einem der vorstehenden Ansprüche, ferner umfassend ein Anzeigefenster (224, 226), das konfiguriert ist, um es einem Benutzer zu ermöglichen, zu sehen, ob die erste und die zweite Betriebsphase jeweils stattgefunden haben oder nicht.

15. System nach einem der vorstehenden Ansprüche, wobei das Arzneimittel eines aus Ketamin, Esketamin, Naloxon oder Sumatriptan ist.

## Revendications

1. Système de délivrance de médicament (100, 200), comprenant :
une tête de distribution (110, 204) comportant un embout (112, 206) conçu pour être positionné dans un nez d'un patient, l'embout ayant une ouverture (108, 208) à l'intérieur de celle-ci ;
un stockage de médicament (102, 202) contenant un médicament à l'intérieur de celui-ci ;
un premier élément d'arrêt (228) ; et
un second élément d'arrêt (232) ; **caractérisé en ce que** :
le stockage de médicament est conçu pour déplacer une première quantité par rapport à la tête de distribution dans une première phase de fonctionnement dans laquelle le médicament est délivré à partir du stockage de médicament et sort par l'ouverture ;
le stockage de médicament est conçu pour déplacer une seconde quantité par rapport à la tête de distribution dans une seconde phase de fonctionnement dans laquelle le médicament est délivré à partir du stockage de médicament et sort par l'ouverture ;
le premier élément d'arrêt est conçu pour arrêter mécaniquement un déplacement du stockage de médicament par rapport à la tête de distribution entre les première et seconde phases de fonctionnement ; et
le second élément d'arrêt est conçu pour arrêter mécaniquement un déplacement du stockage de médicament par rapport à la tête de distribution après la seconde phase de fonctionnement.

2. Système (200) selon la revendication 1, comprenant en outre un accessoire (228) qui comporte le premier élément d'arrêt, qui est conçu pour être accouplé de manière libérable au stockage de médicament pendant la première phase de fonctionnement, et qui est conçu pour être retiré du stockage de médicament entre les première et seconde phases de fonctionnement, dans lequel facultativement l'accessoire a une voie de passage interne s'étendant à travers celui-ci et l'accessoire est conçu pour être accouplé de manière libérable au stockage de médicament pendant la première phase de fonctionnement avec le stockage de médicament s'étendant à travers la voie de passage interne.

3. Système (200) selon la revendication 2, comprenant en outre un corps (212) qui comporte le second élément d'arrêt ; dans lequel :
le second élément d'arrêt comprend une surface extérieure du corps ;
le stockage de médicament est conçu pour déplacer la première quantité par rapport au corps dans la première phase de fonctionnement ; et
le stockage de médicament est conçu pour déplacer la seconde quantité par rapport au corps dans la seconde phase de fonctionnement.

4. Système (200) selon la revendication 1, comprenant en outre un corps (212) qui comporte les premier et second éléments d'arrêt ; dans lequel :
le premier élément d'arrêt comprend une première surface extérieure du corps ;
le second élément d'arrêt comprend une seconde surface extérieure du corps qui est proximale par rapport à la première surface extérieure ;
le stockage de médicament est conçu pour déplacer la première quantité proximalement par rapport au corps dans la première phase de fonctionnement ; et
le stockage de médicament est conçu pour déplacer la seconde quantité proximalement par rapport au corps dans la seconde phase de fonctionnement.

5. Système (300) selon la revendication 1, dans lequel le stockage de médicament (302, 302a) comporte un corps allongé (314, 314a) avec une collerette (304, 304a) s'étendant radialement vers l'extérieur à partir du corps allongé, la collerette étant attachée de façon libérable au corps allongé avec une pluralité de pattes frangibles (316), dans lequel facultativement la pluralité de pattes frangibles sont conçues chacune pour se casser pendant la première phase de fonctionnement.

6. Système (300) selon la revendication 5, comprenant en outre une pièce centrale (306) qui comporte le premier élément d'arrêt ; et
un corps qui comporte le second élément d'arrêt ;
dans lequel le premier élément d'arrêt comprend une partie saillante (320) conçue pour venir en prise avec la collerette ; et
dans lequel le second élément d'arrêt comprend une surface intérieure du corps conçue pour venir en prise avec une surface du stockage de médicament.

7. Système (300) selon la revendication 5, comprenant en outre un corps qui comporte le second élément d'arrêt ; dans lequel :
le stockage de médicament (302a) comporte une seconde collerette (304b) s'étendant radialement vers l'extérieur à partir du corps allongé (314a), la seconde collerette étant attachée de façon libérable au corps allongé avec une seconde pluralité de pattes frangibles qui sont conçues chacune pour se casser pendant la seconde phase de fonctionnement.

8. Système (400) selon la revendication 1, dans lequel le stockage de médicament (402) a des première (404) et seconde (406) rainures formées à l'intérieur de celui-ci ;
le système d'administration de médicaments comporte un corps (412) ayant des première (408) et seconde (410) parties saillantes s'étendant radialement vers l'intérieur à partir de celui-ci ; et
le premier élément d'arrêt comprend la première rainure et la première partie saillante, et le second élément d'arrêt comprend la seconde rainure et la seconde partie saillante.

9. Système (500) selon la revendication 1, dans lequel le stockage de médicament (502) comporte une languette frangible (504) s'étendant radialement vers l'extérieur à partir de celui-ci ; et
le système de délivrance de médicament comporte un corps (506) comportant une partie saillante (508) s'étendant radialement vers l'intérieur à partir de celui-ci qui définit le premier élément d'arrêt, et le corps comporte une surface intérieure qui définit le second élément d'arrêt.

10. Système (600) selon la revendication 1, dans lequel le stockage de médicament (602) est conçu pour être poussé dans une direction axiale en direction de la tête de distribution dans chacune des première et seconde phases de fonctionnement ;
le stockage de médicament a une rainure hélicoïdale formée à l'intérieur de celui-ci ; et
le système de délivrance de médicament comprend en outre une came (606) conçue pour coulisser au sein de la rainure hélicoïdale en réponse à la poussée du stockage de médicament, amenant de ce fait le stockage de médicament à effectuer une rotation par rapport à la tête de distribution.

11. Système (700) selon la revendication 1, dans lequel le stockage de médicament (702) est conçu pour être poussé dans une direction longitudinale en direction de la tête de distribution dans chacune des première et seconde phases de fonctionnement ;
le stockage de médicament a une partie saillante (704) s'étendant à partir de celui-ci ;
le système de délivrance de médicament comprend en outre un corps (706) ayant une piste (708) formée à l'intérieur de celui-ci ;
la partie saillante est conçue pour coulisser longitudinalement au sein de la piste en réponse à la poussée du stockage de médicament, amenant de ce fait la partie saillante du médicament à coulisser axialement dans la piste ;
le stockage de médicament est conçu pour être mis en rotation par rapport à la tête de distribution entre les première et seconde phases de fonctionnement ; et
la partie saillante est conçue pour coulisser axialement au sein de la piste en réponse à la rotation du stockage de médicament.

12. Système (800, 1100) selon la revendication 1, comprenant en outre un corps (810, 1110) avec un manchon (808, 1108) disposé à l'intérieur de celui-ci qui a le stockage de médicament (802, 1102) fixé à celui-ci ;
un actionneur (806, 1106) conçu pour être actionné pour amener le médicament à être délivré par l'ouverture ; et
un ressort de poussée (804, 1104) conçu pour pousser le manchon et le stockage de médicament proximalement en réponse à l'actionnement de l'actionneur ;
dans lequel le premier élément d'arrêt comporte une première surface du manchon ; et
dans lequel le second élément d'arrêt comporte une seconde surface du manchon.

13. Système (900) selon la revendication 1, comprenant en outre un premier actionneur (908) qui comporte le premier élément d'arrêt et qui est conçu pour être actionné pour amener le médicament à être délivré par l'ouverture dans la première phase de fonctionnement ;
un second actionneur (910) qui comporte le second élément d'arrêt et qui est conçu pour être actionné pour amener le médicament à être délivré par l'ouverture dans la première phase de fonctionnement ;
un aimant proximal (906) ; et
un aimant distal (904) conçu pour être attiré proximalement en direction de l'aimant proximal en réponse à l'actionnement du premier actionneur et en réponse à l'actionnement du second actionneur.

14. Système selon l'une quelconque des revendications précédentes, comprenant en outre une fenêtre d'observation (224, 226) conçue pour permettre à un utilisateur de visualiser si chacune des première et seconde phases de fonctionnement s'est ou non produite.

15. Système selon l'une quelconque des revendications précédentes, dans lequel le médicament est l'un parmi kétamine, eskétamine, naloxone et sumatriptan.
